## Europäisches Patentamt

## European Patent Office

## Office européen des brevets

(11) Veröffentlichungsnummer: **0 046 506**
**B1**

(12) # EUROPÄISCHE PATENTSCHRIFT

(45) Veröffentlichungstag der Patentschrift:
**30.05.84**

(51) Int. Cl.³: **C 07 D 461/00**, A 61 K 31/395 //
C07D275/06

(21) Anmeldenummer: **81105633.2**

(22) Anmeldetag: **17.07.81**

(54) **Vincamin-saccharinat und dieses enthaltende Arzneimittel.**

(30) Priorität: **25.08.80 DE 3031953**

(43) Veröffentlichungstag der Anmeldung:
**03.03.82 Patentblatt 82/9**

(45) Bekanntmachung des Hinweises auf die Patenterteilung:
**30.05.84 Patentblatt 84/22**

(84) Benannte Vertragsstaaten:
**AT BE CH DE FR GB IT LI LU NL**

(56) Entgegenhaltungen:
**DE - A - 2 604 044**
**DE - A - 2 725 246**
**FR - A - 2 193 586**
**FR - A - 2 193 587**
**FR - A - 2 284 325**
**US - A - 2 538 645**

**CHEMICAL ABSTRACTS, Band 63, 1965, Spalte 2847h Columbus, Ohio, U.S.A. L.G. BROOKS: "Use of synthetic sweetening agents in pharmaceutical preparations and foods"**

(73) Patentinhaber: **Heinrich Mack Nachf., Postfach 140, D-7918 Illertissen (DE)**

(72) Erfinder: **Räder, Kurt, Dr., Bahnhofstrasse 25, D-7955 Ochsenhausen (DE)**
Erfinder: **Stoss, Peter, Dr., Mozartstrasse 15, D-7918 Illertissen (DE)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

## Beschreibung

Die Erfindung bezieht sich auf ein neues Vincamin-Derivat, nämlich Vincamin-saccharinat, sowie auf diese neue Verbindung enthaltende Arzneimittel.

Vincamin ist ein aus Vinca minor L. isoliertes Alkaloid, das bekanntlich pharmakologische Aktivität als gefässerweiterndes Mittel besitzt und zur Behandlung von Durchblutungsstörungen des Gehirns und neurologischen Störungen verwendet worden ist. Vincamin-Base ist in Wasser und anderen pharmazeutisch annehmbaren Lösungsmitteln praktisch unlöslich und hauptsächlich oral in festen Dosierungsformen, wie Tabletten oder Dragees, verabreicht worden. Vincamin stand auch als Hydrochlorid in fester Form in Ampullen zur Zubereitung verdünnt-wässriger Lösungen mit weniger als etwa 0,5 Gew.-/Gew.-% oder von Suspensionen zur Injektionsverabreichung unter Rekonstitution mit Wasser zur Verfügung.

Ein besonderes Problem bestand darin, eine Vincamin-Form, als ein Salz oder ein anderes Derivat, zu finden, das in einem pharmazeutisch annehmbaren Lösungsmittel hinreichend löslich ist, um eine ausreichende Konzentration des Wirkstoffs in der Lösung zu liefern, so dass die Verabreichung in gelöster Form, vorzugsweise als Tropfen, möglich wird, d.h., die nötige Dosierungsmenge des Wirkstoffs in einem annehmbar kleinen Lösungsmittelvolumen zu ermöglichen. In dieser Hinsicht war ein weiteres, äusserst erhebliches Problem, dass selbst die wenigen Salze oder Komplexe des Vincamins, die sich in pharmazeutisch annehmbaren Lösungsmitteln als relativ löslich erwiesen haben, z.B. Vincamin-citrat oder -tartrat in Glycerin/Äthanol-Gemischen, und die daher eine brauchbare Konzentration des Wirkstoffs in Lösung liefern würden, einen extrem bitteren und unangenehmen Geschmack haben, der unmöglich zu maskieren oder zu überwinden ist, beispielsweise durch Zusatz von Geschmacks- oder Aromastoffen. Der Fehlschlag, eine Form des Vincamins zu finden, die sowohl die gewünschte Löslichkeit als auch annehmbare Geschmackseigenschaften aufweisen würde, hat die Entwicklung medizinisch annehmbarer flüssiger Arzneimittel zur oralen Verabreichung, insbesondere von Lösungen mit einer ausreichenden Konzentration des aktiven Wirkstoffs, die als Tropfen verabreicht werden können, verhindert. Es bestand daher ein erheblicher Bedarf an einem Derivat des Vincamins, das relativ hohe Löslichkeit in einem pharmazeutisch annehmbaren Lösungsmittel aufweist, aber keine bitteren oder anderweitig unangenehmen Geschmackseigenschaften hat.

Aus der FR-A-2 193 587 und der FR-A-2 193 586 ist es bekannt, den bitteren Geschmack von Vincamin dadurch zu maskieren, dass man dieses in das Laurylsulfat bzw. das Dioctylsulfosuccinat überführt. Aus der DE-A1-2 604 044 ist es bekannt den bitteren Geschmack pharmazeutischer Wirkstoffe durch Zumischung von in Wasser unlöslichen Substanzen mit einem hohen Molekulargewicht und ein besonderes Granulierungsverfahren zu maskieren. Weiterhin ist aus der letztgenannten Druckschrift sowie aus «The Chemist and Drugist» 183 (4445) 421–3 (1965) bekannt, Saccharin als Süssungsmittel in der Pharmazie zu verwenden.

Es wurde nun gefunden, dass die neue Verbindung, Vincamin-saccharinat, in Glycerin/Äthanol-Gemischen ausreichend löslich ist, um brauchbare Konzentrationen des aktiven Bestandteils in Lösung zu liefern, und zugleich Geschmackseigenschaften besitzt, die für flüssige, für orale Verabreichung geeignete Arzneimittel annehmbar sind. Somit betrifft die Erfindung die neue Verbindung Vincamin-Saccharinat sowie auch pharmazeutische Zubereitungen von Vincamin-saccharinat und einem pharmazeutisch annehmbaren Verdünnungsmittel oder Träger. Insbesondere enthalten erfindungsgemässe pharmazeutische Zubereitungen 0,5 bis 3 Gew./Gew.-% Vincamin-saccharinat, gelöst in einem Lösungsmittel aus etwa 10 bis 90 Gewichtsteilen Glycerin und etwa 90 bis 10 Gewichtsteilen Äthanol. Ein besonders bevorzugtes Arzneimittel enthält etwa 3 Gew.-/Gew.-% Vincamin-saccharinat, gelöst in einem Gemisch aus etwa 70 Gewichtsteilen Glycerin und etwa 30 Gewichtsteilen Äthanol.

Die erfindungsgemässe neue Verbindung, Vincamin-saccharinat, wird durch Umsetzen von Vincamin-Base und Saccharin (1,2-Benzisothiazol-3(2H)-on-1,1-dioxid) hergestellt. Die Umsetzung kann unter Lösen von Vincamin-Base in einer Säure, wie Salzsäure, Schwefelsäure, Phosphorsäure, Milchsäure, Zitronensäure, Ketoglutarsäure, Maleinsäure, Malonsäure, Weinsäure und anschliessende Zugabe einer äquimolaren Menge eines Saccharinats in wässriger Lösung, vorzugsweise eines Alkalimetallsaccharinats, wie Natrium- oder Kaliumsaccharinat, erfolgen. Vincamin-saccharinat bildet sich als Niederschlag und kann leicht abgetrennt werden, z.B. durch Filtrieren, und, wenn gewünscht, gereinigt werden, z.B. durch Wiederauflösen des Vincaminsaccharinats unter Erwärmen des Niederschlags in Aceton unter Rückfluss und anschliessendes Konzentrieren der Lösung sowie Zugabe von Wasser, worauf das Vincamin-saccharinat wieder ausfällt.

Nach einer anderen Arbeitsweise kann Vincamin-Base und Saccharin zu einem Niederalkylalkohol, vorzugsweise Methanol, gegeben werden, worauf Wasser zugesetzt und auf eine Temperatur von 50 bis 55 °C erwärmt wird. Beim Abkühlen, z.B. auf etwa 0 °C, wird Vincamin-saccharinat als kristalliner Feststoff erhalten.

Das Vincamin-saccharinat, hergestellt, wie oben beschrieben, hat einen Schmelzpunkt unter Zersetzung im Bereich von 189 bis 194 °C, je nach der Ausgangstemperatur und der beim Messen des Schmelzpunkts angewandten Aufheizgeschwindigkeit.

Ohne an eine besondere Feststellung oder Theorie hinsichtlich der Art des gebildeten, wie vorstehend beschriebenen Produkts gebunden sein zu wollen, wird angenommen, dass Vincamin-saccharinat tatsächlich ein Salz ist. Das wie oben beschrieben erhaltene Vincamin-saccharinat liegt vermutlich in einer hydratisierten Form vor, wobei der Wassergehalt im Bereich von 0,5 bis 1,0 Mol Wasser pro Mol freies Vincamin-saccharinat liegt. Wenn gewünscht, kann wasserfreies Vincamin-saccharinat erhalten werden, indem z.B. die hydratisierte Form auf etwa 50 °C unter Hochvakuum erwärmt wird. Die wasserfreie Verbindung ist jedoch hygroskopisch und kehrt in der Atmosphäre wieder in die hydratisierte Form zurück. Der hier verwendete Ausdruck Vincamin-saccharinat soll das durch Kombination äquimolarer Mengen an Vincamin oder dessen Salzen mit Saccharin oder Saccharinaten gebildete Produkt umfassen, unabhängig von der genauen physikalischen Natur oder Struktur des Produkts.

Vincamin-saccharinat ist als Gefässerweiterer zur Behandlung cerebral-zirkulatorischer Zustände, wie cerebraler Hypoxie, und damit verbundener neurologischer Störungen, wie Aphasie, Apraxie und Agnosie, in Warmblütern, insbesondere beim Menschen, brauchbar. Das Vincamin-saccharinat wird im allgemeinen oral in Dosen zwischen 0,8 und 1,3 mg/kg/Tag, vorzugsweise etwa 1 mg/kg/Tag, entweder in einer Einzeldosis oder vorzugsweise in zwei bis vier Dosen pro Tag verabreicht. Solche Dosierungen liegen erheblich unter den $LD_{50}$-Werten, bestimmt für Vincamin-saccharinat in Mäusen, nämlich 2900 mg/kg p.o. und 560 mg/kg i.p.

Während Vincamin-saccharinat, wenn gewünscht, oral in Form fester pharmazeutischer Zubereitungen mit Vincamin-saccharinat und einem pharmazeutisch annehmbaren festen Träger oder Füllstoff in Dosierungsformen wie Tabletten, Kapseln, Dragees oder Pulvern, verabreicht werden kann, machen es die Löslichkeits- und Geschmackseigenschaften besonders geeignet zur Verabreichung in flüssigen pharmazeutischen Mitteln. Insbesondere pharmazeutische Mittel mit 0,5 bis 3 Gew./Gew.-%, vorzugsweise 2 bis 3, insbesondere etwa 3% Vincamin-saccharinat, gelöst in einem Lösungsmittelgemisch aus 10 bis 90 Gewichtsteilen Glycerin und 90 bis 10 Gewichtsteilen Äthanol, insbesondere 70 Teilen Glycerin und 30 Teilen Äthanol, sind bevorzugt, da solche Lösungen eine genügende Konzentration des aktiven Bestandteils in Lösung enthalten, um die nötige Dosismenge des Wirkstoffs in einem begrenzten Flüssigkeitsvolumen zu liefern, und werden so leicht oral, insbesondere als Tropfen, verabreicht. So liefert z.B. die Verabreichung zwischen 0,5 und 1 ml einer Lösung, die zwischen 2 und 3 Gew./Gew.-% enthält, die nötige Dosismenge an aktivem Bestandteil bei oraler Verabreichung in zwei bis vier Dosen pro Tag.

Äthanol für die Verwendung im Lösungsmittelgemisch der oben beschriebenen pharmazeutischen Mittel kann Äthanol von Handelsqualität sein, d.h. 92,3 Gew./Gew.-% Äthanol, Rest Wasser, ist aber bevorzugt absolutes Äthanol (99,9 Gew./Gew.-%). Glycerin für die erfindungsgemässe Verwendung kann 85 gew./gew.-%ig sein, Rest Wasser, ist aber vorzugsweise wasserfreies Glycerin.

Wenn gewünscht, können weitere herkömmliche Bestandteile, wie färbende Mittel oder Farbstoffe, Konservierungsmittel, Geschmacks- bzw. Aromastoffe, den oben beschriebenen pharmazeutischen Mitteln in Lösungsform zugesezt werden.

Neben den vorstehend beschriebenen pharmazeutischen Mitteln in Lösungsform sind auch solche in flüssiger Form, die Suspensionen von Vincamin-saccharinat in einem pharmazeutisch annehmbaren flüssigen Medium aufweisen, von Wert für die orale Verabreichung, und zwar aufgrund der überlegenen Geschmackseigenschaften von Vincamin-saccharinat. Solche Suspensionen können 0,5 bis 10 Gew./Gew.-% Vincamin-saccharinat in einem wässrigen oder organischen Medium, z.B. in wässrigen Sorbitlösungen, zusammen mit, sofern gewünscht, weiteren Bestandteilen, wie Geschmacks- bzw. Aromamitteln, emulgierenden oder dispergierenden Mitteln, färbendem Material oder Farbstoffen, Konservierungsmittel enthalten.

Die Erfindung wird durch die folgenden Beispiele weiter veranschaulicht. Natürlich wird sie durch die speziellen Einzelheiten dieser Beispiele nicht begrenzt.

Beispiel 1

0,1 Mol (35,44 g) Vincamin-Base und 0,1 Mol (15,0 g) Weinsäure werden in 1,75 l Wasser gelöst. Unmittelbar nach dem Lösen des Gemischs wird 0,1 Mol (20,5 g) Natrium-saccharinat, in 100 ml Wasser gelöst, unter Rühren bei Raumtemperatur zugesetzt. Nach einstündigem Rühren wird der Niederschlag abfiltriert und in Aceton auf Rückfluss erwärmt.

Die filtrierte Lösung wird durch Einengen auf etwa 100 ml konzentriert, und dann werden 700 ml Wasser zugesetzt. Nach 24-stündigem Stehen in einem Kühlschrank wird der Niederschlag abfiltriert und zu Vincamin-saccharinat, Schmp. 189,4 °C, getrocknet (Ausbeute – nach Aufarbeiten der Mutterlauge – 50,8 g = 95%).

Beispiel 2

3,0 l Methanol, 354,4 g Vincamin und 183,2 g Saccharin werden in einen 6,0 l-Kolben, ausgestattet mit Rührer und Thermometer, gebracht. Unter Rühren werden 300 ml entionisiertes Wasser zugesetzt, und der Inhalt wird auf 50 bis 55 °C erwärmt. Es ergibt sich eine klare Lösung, die filtriert und unter weiterem Rühren auf 0 °C heruntergekühlt wird. Das kristalline Produkt wird dann abfiltriert und mit einer (0 °C) kalten Lösung aus 0,5 l Methanol und 50 ml entionisiertem Wasser gewaschen. Das Produkt wird bei 40 °C unter Wasserstrahlvakuum in einem Ofen getrocknet. Ausbeute 490 g Vincamin-saccharinat, Schmp. 192 °C (unter Zers.; Schmelzpunktsapparatur Typ Büchi 510, Anfangstemperatur 180 °C, Aufheizgeschwindigkeit 2 °C/min).

Elementaranalyse, %:
gef.:
ber. für $C_{28}H_{31}N_3O_6S \cdot 1/2H_2O$:
ber. für $C_{28}H_{31}N_3O_6S \cdot H_2O$:

C 60,86;  H 5,84;  N 7,68;  S 5,71
C 61,52;  H 5,90;  N 7,68;  S 5,86
C 60,52;  H 5,98;  N 7,56;  S 5.77.

Die wasserfreie Form des Vincamin-saccharinats entsteht durch Erwärmen des Produkts des Beispiels auf 50 °C unter Hochvakuum.

Elementaranalyse, %:
gef.:
ber. für $C_{28}H_{31}N_3O_6S$:

C 62,39;  H 5,90;  N 7,71
C 62,55;  H 5,81;  N 7,81

Beispiel 3

3,0 g Vincamin-saccharinat werden unter Rühren bei Raumtemperatur in 97 g eines Gemischs aus 70 Gewichtsteilen Glycerin («Europäisches Arzneibuch III»), 85 Gew./Gew.-%, und 30 Gewichtsteilen 99,9%igen Äthanols, gelöst. Nach etwa 4 h wird die Lösung filtriert. Die anfallende Lösung eignet sich für die orale Verabreichung in Form von Tropfen.

Beispiel 4

0,21 g p-Hydroxybenzoesäuremethylester und 0,09 g p-Hydroxybenzoesäurepropylester wurden unter Erwärmen in 35 g Wasser gelöst und mit 35 g einer 70 gew.-%igen wässrigen Sorbitlösung gemischt. 9,085 g Vincamin-saccharinat wurden in diesem Gemisch suspendiert und 0,05 g «Polysorbat 60»® zugesetzt. Die Suspension wurde auf einen pH-Wert zwischen 6,5 und 7,5 durch Zugabe von Natriumbicarbonatlösung eingestellt und mit Wasser auf 100 g verdünnt, um ein Arzneimittel in für die orale Verabreichung geeigneter Suspensionsform zu ergeben.

**Patentansprüche für die Vertragsstaaten: BE, CH, DE, FR, GB, IT, LI, LU, NL**

1. Vincamin-saccharinat.

2. Vincamin-saccharinat in einem pharmazeutisch annehmbaren Träger oder Verdünnungsmittel.

3. Arzneimittel mit 0,5 bis 3 Gew./Gew.-% Vincamin-saccharinat, gelöst in einem Lösungsmittel, bestehend aus 10 bis 90 Gewichtsteilen Glycerin und 90 bis 10 Gewichtsteilen wasserfreiem Äthanol.

4. Arzneimittel mit 3 Gew.-/Gew.-% Vincamin-saccharinat, gelöst in einem Lösungsmittel, bestehend aus 70 Teilen Glycerin und 30 Teilen Äthanol.

**Patentansprüche für den Vertragsstaat: AT**

1. Verfahren zur Herstellung von Vincamin-Saccharinat, dadurch gekennzeichnet, dass Vincamin-Base oder eins ihrer Säureadditionssalze mit Saccharin oder einem Alkalimetallsaccharinat umgesetzt wird, wobei die Vincamin-Base mit Saccharin in einer wässrigen, nieder-Alkylalkohol-Lösung und ein Vincamin-Säureadditionssalz mit einem Alkalimetallsaccharinat in wässriger Lösung umgesetzt werden.

2. Verfahren gemäss Anspruch 1, dadurch gekennzeichnet, dass die Vincamin-Base mit Saccharin in wässrigem Methanol bei einer Temperatur von 50–55 °C umgesetzt wird.

3. Verfahren gemäss Anspruch 1, dadurch gekennzeichnet, dass ein Vincamin-Säureadditionssalz mit Natrium- oder Kaliumsaccharinat in wässriger Lösung bei Raumtemperatur umgesetzt wird.

4. Verfahren gemäss Anspruch 3, dadurch gekennzeichnet, dass das Vincaminsalz Vincamin-tartrat ist.

**Revendications pour les Etats contractants: BE, CH, DE, FR, GB, IT, LI, LU, NL**

1. Saccharinate de vincamine.

2. Saccharinate de vincamine dans un support ou diluant pharmaceutiquement acceptable.

3. Médicament contenant 0,5 à 3% en poids/poids de saccharinate de vincamine, en solution dans un solvant formé de 10 à 90 parties en poids de glycérine et de 90 à 10 parties en poids d'éthanol anhydre.

4. Médicament contenant 3% en poids/poids de saccharinate de vincamine en solution dans un solvant formé de 70 parties de glycérine et de 30 parties d'éthanol.

**Revendications pur l'Etat contractant: AT**

1. Procédé de production du saccharinate de vincamine, caractérisé en ce qu'on fait réagir de la vincamine base ou l'un de ses sels d'addition d'acides avec la saccharine ou avec un saccharinate de métal alcalin, en conduisant alors la réaction de la vincamine base avec la saccharine dans une solution aqueuse d'un alcool alkylique inférieur et la réaction d'un sel d'addition d'acide de la vincamine avec un saccharinate de métal alcalin en solution aqueuse.

2. Procédé suivant la revendication 1, caractérisé en ce que la vincamine base est amenée à réagir avec la saccharine dans du méthanol aqueux à une température de 50 à 55 °C.

3. Procédé suivant la revendication 1, caractérisé en ce qu'on fait réagir un sel d'addition d'acide de la vincamine avec le saccharinate de sodium ou de potassium en solution aqueuse à la température ambiante.

4. Procédé suivant la revendication 3, caractérisé en ce que le sel de vincamine est le tartrate de vincamine.

**Claims for the Contracting States: BE, CH, DE, FR, GB, IT, LI, LU, NL**

1. Vincamine saccharinate.

2. Vincamine saccharinate in a pharmaceutically acceptable carrier or diluent.

3. Drug comprising from 0.5 to 3 percent by weight vincamine saccharinate dissolved in a solvent consisting of 10 to 90 parts by weight glycerol and 90 to 10 parts by weight absolute ethanol.

4. Drug comprising 3 percent by weight vincamine saccharinate dissolved in a solvent consisting of 70 parts glycerol and 30 parts ethanol.

**Claims for the Contracting State: AT**

1. Process for the preparation of vincamine saccharinate characterized in that vincamine base or one of its acid addition salts is reacted with saccharine or an alkali metal saccharinate, whereby the vincamine base is reacted with saccharine in an aqueous, lower alkyl alcohol solution and a vincamine acid addition salt is reacted with a alkali metal saccharinate in an aqueous solution.

2. Process according to claim 1 characterized in that the vincamine base is reacted with saccharine in aqueous methanol at a temperature of 50–55 °C.

3. Process according to claim 1 characterized in that a vincamine acid addition salt is reacted with sodium or potassium saccharinate in aqueous solution at room temperature.

4. Process according to claims 3 characterized in that the vincamine salt is vincamine tartrate.